# EUROPEAN PATENT APPLICATION

(11) **EP 1 658 861 A1**
(43) Date of publication of application: **24.05.2006**
(21) Application number: 04772314.3
(22) Date of filing: 27.08.2004
(51) Int. Cl.: A61K 47/30, A61K 9/70, A61L 15/07, C09J 4/00, C08G 18/83

(54) **ACTIVE ENERGY RADIATION HARDENABLE SKIN PLASTER COMPOSITION AND SKIN PLASTER**

(30) Priority: 27.08.2003 JP 2003303746
(71) Applicant: Toagosei Co., Ltd, Minato-ku, Tokyo 105-8419 (JP)
(72) Inventor: ITO, Kenji, c/o Taogosei Co., Ltd., Minato-ku, Nagoya-shi, Aichi 455-0027 (JP); KAMIYA, Daisuke, c/o Taogosei Co., Ltd., Minato-ku, Nagoya-shi, Aichi 455-0027 (JP)
(74) Representative: Pohlmann, Eckart
(86) International application number: PCT/JP2004/012358
(87) International publication number: WO 2005/021042

(57) **Abstract**

[Problems] A composition curable with active energy beams for use in skin patches is provided, which is liquid at ordinary temperature, has a practical crosslinking property or curability in the absence of photopolymerization initiators even when irradiated with visible or ultraviolet light, and provides cured films that are not discolored and excellent in various pressure-sensitive adhesive performances and have appropriate pressure-sensitive adhesiveness so as not to cause pain or damage to the horny layer in the patch-applied area even when patches are repeatedly applied and peeled off, as well as excellent water resistance. [Means for solving problems] The composition curable with active energy beams for use in skin patches is liquid at ordinary temperature and comprises (A) a compound having two or more maleimide groups and (B) an oil component, or water or a water-soluble compound.

## Description

### Technical Field

The present invention relates to a composition curable with active energy beams for use in skin patches, which is composed of a compound containing two or more specific maleimide groups, and also relates to a skin patch having a pressure-sensitive adhesive layer formed of the above described composition. They can be utilized with admiration in the relevant technical fields.

### Background Art

Patches are used for the purpose of protecting injured parts of skin surface, the purpose of having medicinal ingredients for treatment of disease absorbed through the skin to deliver them into living bodies, and other purposes. Specifically, they are known as first aid plasters, magnetic plasters, surgical tapes, wound-healing dressing materials (dressings), athletic tapes, tape preparations, plasters, cataplasms and others.

Performances required for the patches are such that the patches are excellent in adhesiveness to skin, no pain or exfoliation of horny layer is caused when the patches are peeled off, and the patches are low in skin irritation and high in safety since they are applied directly to skin.

As pressure-sensitive adhesive components for use in patches, there are commonly used solvent based pressure-sensitive adhesives that are composed of acrylic polymers and rubber-based resins dissolved in organic solvents (for example, Patent Documents 1 and 2).

However, there is a fear that the solvent based pressure-sensitive adhesives might have an adverse environmental effect due to organic solvents evolved when processed into patches, and additionally have an adverse effect on safety due to residual solvent contained in coating films of the pressure-sensitive adhesives. Thus, in these years, pressure-sensitive adhesives using no organic solvents come to be demanded.

As an alternative for solvent based pressure-sensitive adhesives, active energy beam-curable pressure-sensitive adhesives, which are curable with active energy beams such as ultraviolet light and visible light, have been investigated because they are easy to handle owing to low viscosity, adaptable to coating devices that have been used for the solvent based adhesives, and excellent in water resistance.

Generally, raw materials of compositions curable with active energy beams including pressure-sensitive adhesives curable with active energy beams are not crosslinked or cured by themselves, and hence it is necessary to add a photopolymerization initiator or a photosensitizer (hereinafter, these are collectively referred to as photopolymerization initiator or the like) .

The greater the addition amount of the photopolymerization initiator or the like is, the quicker the curing is; and thus, the addition amount thereof is apt to be increased. However, the photopolymerization initiator or the like includes a compound having an aromatic ring so as to absorb light efficiently, thereby causing a problem of yellowing of the resultant cured films.

Additionally, the photopolymerization initiator or the like is usually composed of a low molecular weight compound so as to efficiently initiate polymerization reaction. However, when the composition is irradiated with an active energy beam, the temperature is elevated owing to polymerization heat, and thus the low molecular weight photopolymerization initiator or the like, which is high in vapor pressure, causes problems such that malodor is generated markedly at the time of curing, thereby causing a problem of working environment, or the obtained products are polluted. Additionally, there are problems in that decomposition products including an unreacted photopolymerization initiator and the like remain in the cured films, and hence, when the cured films are subjected to light or heat, there often occurs a problem such that the cured films turn yellow, or unfavorable odor is generated.

Furthermore, in the case where the composition is applied to patches, when the cured films are made wet with water, or the cured films are touched with sweat and the like excreted from human body, the unreacted photopolymerization initiator or the like bleeds out in a large quantity, and hence there occurs a problem of safety and health.

For the purpose of overcoming the drawbacks of the active energy beam-curable compositions that contain the aforementioned photopolymerization initiator or the like, there have been investigated compositions containing neither photopolymerization initiator nor the like but curable by irradiation with active energy beams (for example, Patent Documents 3 to 6).
· Patent Document 1: Japanese Patent Laid-Open No. H7-69870 (Claims)
· Patent Document 2: Japanese Patent Laid-Open No. H11-12163 (Claims)
· Patent Document 3: Japanese Patent Laid-Open No. H11-124403 (Claims)
· Patent Document 4: Japanese Patent Laid-Open No. H11-124404 (Claims)
· Patent Document 5: Japanese Patent Laid-Open No. 2001-219508 (Claims)
. Patent Document 6: Japanese Patent Laid-Open No. 2001-220567 (Claims)

### Disclosure of the Invention

### Problems to be solved by the Invention

It is disclosed in Patent Documents 3 and 4 that compositions which contain compounds having maleimide groups can be used as a pressure-sensitive adhesive among twenty-odd types of applications disclosed therein. They only disclose the pressure-sensitive adhesive equally to the other disclosed types of applications, but naturally do not disclose the use as skin patches at all.

On the other hand, compositions that contain compounds having maleimide groups disclosed in Patent Documents 5 and 6 are both adhesives the cured coating films of which have no tackiness or are poor in tackiness, so that they are hardly usable as pressure-sensitive adhesives. Additionally, the maleimide group-containing compound disclosed in Patent Document 6 is crystalline with a melting point of 40°C or above, and is not liquid at ordinary temperature, so that it is particularly poor in tackiness and hardly usable as a pressure-sensitive adhesive.

The present inventors have made intensive researches for the purpose of finding a composition curable with active energy beams for use in skin patches which is liquid at ordinary temperature, has a practical crosslinking property or curability in the absence of photopolymerization initiators even when irradiated with visible or ultraviolet light, and provides cured films that do not change color but have appropriate pressure-sensitive adhesiveness so as to cause neither pain in the patch-applied area nor damage to the horny layer of the patch-applied area even when patches are repeatedly applied to and peeled off the skin as well as excellent various pressure-sensitive adhesive performances, and are also excellent in water resistance.

### Means that solve the Problems

The present inventors have performed various investigations for the purpose of solving the above described problems, and consequently have accomplished the present invention by finding out that a composition for use in skin patches which contains a compound having two or more maleimide groups and an oil component, or water or a water-soluble compound can solve the above described problems.

Hereinafter, detailed description of the present invention will be made.

### 1. (A) Compounds having two or more maleimide groups

The composition curable with active energy beams for use in skin patches according to the present invention contains, as an indispensable compound, a compound having two or more maleimide groups (hereinafter, also referred to as a maleimide compound).

Various functional groups can be used as the maleimide groups in the component (A), and the maleimide group is preferably those represented by the following formulas (1) to (4). 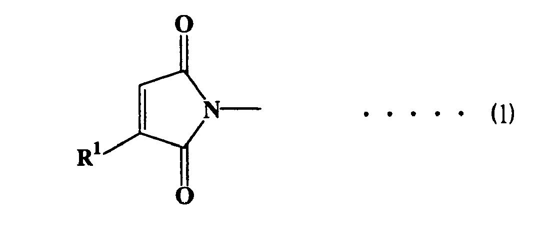 (In formula (1), R¹ represents an alkyl group, an aryl group, an arylalkyl group or a halogen atom.) 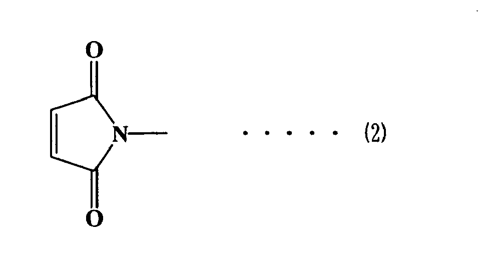 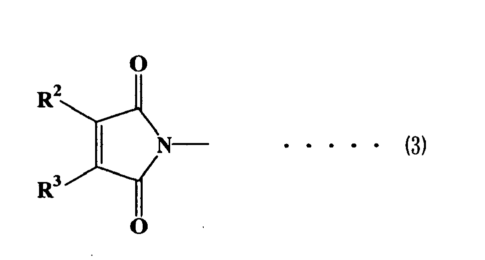 (In formula (3), R² and R³ each represent an alkyl group, an aryl group, an arylalkyl group or a halogen atom.) 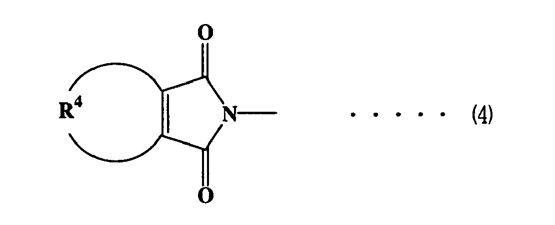 (In formula (4), R⁴ represents a propylene or butylene group which may have substituents.)

In above formulas (1) and (3), the alkyl groups, as R¹ to R³, each are preferably a group having 4 or less carbon atoms, and particularly preferably a methyl group. Examples of an aryl group include a phenyl group. Examples of an arylalkyl group include a benzyl group.

Preferable as R¹ to R³ among these groups is an alkyl group, more preferable is an alkyl group having 4 or less carbon atoms, and particularly preferable is a methyl group.

In above formula (4), as the propylene or butylene group as R⁴ which may have substituents, a butylene group is preferable from the viewpoint of being easily available and being excellent in various adhesive performances. In the case of the propylene or butylene group having a substituent, the substituent is preferably an alkyl group, and more preferable is a methyl group. Examples of the propylene or butylene group having a substituent include -CH₂CH(CH₃)CH₂CH₂-.

The component (A) preferably comprises as the indispensable compound a maleimide compound selected from the following component (I) or the following component (II) since they are excellent in crosslinking or curing (hereinafter these will be collectively referred to as "curing") performance and the resultant cured film is excellent in water resistance.
(I): A compound (hereinafter referred to as compound 1) having two or more maleimide groups represented by the above formula (1).
(II): (a) A compound (hereinafter referred to as compound 2) having two or more maleimide groups represented by the above formula (2) and (b) a compound (hereinafter referred to as compound 3) having two or more maleimide groups represented by the above formula (3) or/and a compound (hereinafter referred to as compound 4) having two or more maleimide groups represented by the above formula (4).

The component (A) used in the present invention contains the maleimide groups, and accordingly, irradiation with an active energy beam leads to dimerization of the maleimide groups, so that the compound molecules are crosslinked to each other. Additionally, also in the case where curing is made with the aid of ultraviolet light or visible light, the maleimide groups can cause the dimerization reaction with the aid of irradiation with ultraviolet light or visible light without blending of a photopolymerization initiator or the like, or with blending of a small amount of a photopolymerization initiator or the like.

Additionally, the component (A) in the present invention is preferably liquid at ordinary temperature; accordingly, handling including coating operation comes to be easy. On the contrary, compounds which are solid at ordinary temperature are not easy to handle, and additionally, lead to a high elastic modulus of the cured film and thus to insufficient adhesive performance. Here, it should be noted that ordinary temperature referred to in the present invention means 25°C.

The molecular weight of the component (A) is preferably 1,000 to 20,000 in terms of number average molecular weight, more preferably 2,000 to 10,000, and further preferably 2,000 to 8,000. When the number average molecular weight is less than 1,000, the pressure-sensitive adhesive strength and tackiness of the cured film are lowered as the case may be, while when the number average molecular weight exceeds 20,000, the viscosity of the composition becomes too high, and coatability is lowered as the case may be and the composition does not become liquid at ordinary temperature even with the component (B) blended therein as the case may be.

Here, it should be noted that the number average molecular weight referred to in the present invention is a value converted from the molecular weight measured by gel permeation chromatography (hereinafter abbreviated as GPC) by use of tetrahydrofuran as solvent with reference to the molecular weight of polystyrene.

Additionally, when the component (A) is a water-soluble polymer, the number average molecular weight is a value converted from the molecular weight measured by GPC by use of a phosphate buffer solution as solvent with reference to the molecular weight of polyethylene oxide.

As the component (A) to be used in the present invention, various compounds can be used as far as the compounds have maleimide groups. As the component (A), compounds prepared by means of various methods can be used; however, the following 3 types of compounds are preferable because of easiness in preparation.
(1) An addition reaction product (hereinafter referred to as compound (1)) between a prepolymer having two or more isocyanate groups at terminals and a compound having a maleimide group and an active hydrogen group.
(2) An esterification reaction product (hereinafter referred to as compound (2)) between a prepolymer having two or more carboxyl groups at terminals and a compound having a maleimide group and an active hydrogen group.
(3) An esterification reaction product (hereinafter referred to as compound (3)) between a prepolymer having two or more hydroxy groups at terminals and a carboxylic acid having a maleimide group.

Now, description will be made below of the compounds (1) to (3).

### 1-1. Compound (1)

The compound (1) is an addition reaction product between a prepolymer (hereinafter simply referred to as urethane prepolymer) having two or more isocyanate groups at terminals and a compound (hereinafter simply referred to as a maleimide/active hydrogen compound) having a maleimide group and an active hydrogen group, and is prepared by reacting 2 or more moles of a maleimide/active hydrogen compound with 1 mole of a urethane prepolymer.

Description will be made below of the urethane prepolymer and the maleimide/active hydrogen compound.

### A) Urethane prepolymer

As the urethane prepolymer, various compounds can be used as far as the compounds each have two or more isocyanate groups at terminals of the molecule thereof.

The urethane prepolymer includes a reaction product between a polyol having two or more hydroxy groups (hereinafter simply referred to as polyol) and a polyisocyanate having two or more isocyanate groups (hereinafter simply referred to as polyisocyanate), and the like.

### a1) Polyol

The polyol includes polyether polyol, polyester polyol, polymer polyol prepared from radically polymerizable monomers, and the like. Among these, polyether polyol and polyester polyol are preferable because the resultant cured films are low in viscosity, and the resultant pressure-sensitive adhesives are excellent in water resistance as well as in pressure-sensitive adhesiveness.

As the polyol, two or more types of polyols can be used as required.

### a1-1) Polyether polyol

The polyether polyol includes polyalkyleneglycols such as polyethyleneglycol, polypropyleneglycol, polybutyleneglycol and polytetramethyleneglycol; ethylene oxide-modified products, propylene oxide-modified products, butylene oxide-modified products and tetrahydrofuran-modified products of alkyleneglycols such as ethyleneglycol, propanediol, propyleneglycol, tetramethyleneglycol, pentamethyleneglycol, hexanediol, neopentylglycol, glycerin, trimethylolpropane, pentaerythritol, diglycerin, ditrimethylolpropane and dipentaerythritol; copolymers of ethylene oxide and propylene oxide, copolymers of propyleneglycol and tetrahydrofuran and copolymers of ethyleneglycol and tetrahydrofuran; hydrocarbon based polyols such as polyisopreneglycol, hydrogenated polyisopreneglycol, polybutadieneglycol and hydrogenated polybutadieneglycol; and polytetramethylenehexaglycerylether (tetrahydrofuran-modified products of hexaglycerin).

Preferable among these polyether polyols are the polyether polyols comprising propylene oxide unit as the indispensable unit. In this way, the resultant maleimide compound can be made liquid.

As the polyether polyol comprising propylene oxide unit as the indispensable unit, may be used a polyether polyol comprising an ethylene oxide unit in addition to the propylene oxide unit, in which proportion of propylene oxide unit can be controlled depending upon whether an oil component, or water or a water-soluble compound is blended as the component (B).

Referring to the polyether polyol comprising both propylene oxide unit and ethylene oxide unit, the proportion of propylene oxide unit is preferably high when the oil component blended; more concretely, the proportion of propylene oxide unit is preferably 30 to 100% by mass, and more preferably 60 to 100% by mass, in relation to the total amount of propylene oxide unit and ethylene oxide unit. When water or a water-soluble compound (hereinafter these will be collectively referred to as the "aqueous ingredient") is blended, the concrete proportion of polypropylene oxide unit is preferably 1 to 90% by mass, and more preferably 15 to 90% by mass.

### a1-2) Polyester polyol

A polyester polyol is a random condensation copolymer of a polycarboxylic acid and a polyhydric alcohol. Among the polyester polyols, an aliphatic polyester polyol is preferable because of excellent curability by active energy beams of the resultant composition.

In this connection, as the polycarboxylic acid, various polycarboxylic acids can be used as far as the polycarboxylic acids each have two or more carboxyl groups in molecule thereof. Specific examples include succinic acid, adipic acid, pimelic acid, suberic acid, azelaic acid, sebacic acid, dodecanedioic acid, icosanedioic acid, 2,6-naphthalene dicarboxylic acid, trimellitic acid, glutaric acid, 1,9-nonane dicarboxylic acid, 1,10-decane dicarboxylic acid, malonic acid, fumaric acid, 2,2-dimethylglutaric acid, 1,3-cyclopentane dicarboxylic acid, 1,4-cyclohexane dicarboxylic acid, 1,3-cyclohexane dicarboxylic acid, itaconic acid, maleic acid, 2,5-norbornane dicarboxylic acid, 1,4-terephthalic acid, 1,3-terephthalic acid, dimeric acid and paraoxybenzoic acid.

Among these, aliphatic dicarboxylic acids are preferable, and adipic acid and sebacic acid are more preferable.

Two or more types of the polycarboxylic acids can be used in combination, as required.

As the polyhydric alcohol, various compounds can be used as far as the compounds each have two or more hydroxy groups in molecule thereof. Specific examples thereof include butylethylpropanediol, 2,4-diethyl-1,5-pentanediol, 3-methyl-1,5-pentanediol and polyethyleneglycol, ethyleneglycol, diethyleneglycol, triethyleneglycol, tetraethyleneglycol, 1,3-propanediol, 2-butyl-2-ethyl-1,3-propanediol, 1,2-butanediol, 1,3-butanediol, 1,4-butanediol, 1,5-pentanediol, 1,6-hexanediol, 1,7-heptanediol, 1,2-octanediol, 1,8-octanediol, 1,9-nonanediol, 1,2-decanediol, 1,10-decanediol, 2,2-dimethyl-1,3-propanediol, 2,2,4-trimethyl-1,6-hexanediol, 1,3-cyclohexanedimethanol and 1,4-cyclohexanedimethanol, dimeric acid diol and 2-methyl-1,8-octanediol.

Among these, aliphatic diols are preferable, and furthermore butylethylpropanediol, 2,4-diethyl-1,5-pentanediol, 3-methyl-1,5-pentanediol, dimeric acid diol and 2-methyl-1,8-octanediol are more preferable because the resultant compositions are low in viscosity and excellent in pressure-sensitive adhesiveness and water resistance.

Two or more types of polyhydric alcohols can be used in combination, as required.

The method for preparing the polyester polyol can be in accordance with general esterification methods, and examples of the method include a method in which in the presence of a catalyst, a polycarboxylic acid and a polyhydric alcohol are heated under stirring, and the like.

As the catalyst, catalysts usually used in the esterification reaction can be used, and examples thereof include base catalysts, acid catalysts and metal alkoxides. Examples of the base catalyst include metal hydroxides such as sodium hydroxide and potassium hydroxide, and amines such as triethylamine, N,N-dimethylbenzylamine and triphenylamine. Examples of the acid catalyst include sulfuric acid and paratoluenesufonic acid. As the metal alkoxide, alkoxides of titanium, tin or zirconium are preferable. Specific examples of these metal alkoxides include tetraalkyl titanates such as tetrabutyl titanate; tin alkoxides such as dibutyltin oxide and monobutyltin oxide; and zirconium alkoxides such as zirconium tetrabutoxide and zirconium isopropoxide.

The reaction temperature and time in the esterification reaction may be appropriately set according to purposes. The reaction temperature is preferably 80 to 220°C.

As the aliphatic polyester polyols, commercially available ones can be used; examples thereof include "Kuraray Polyol P-5010" or "Kuraray Polyol P-5050" manufactured by Kuraray Co., Ltd., "KYOWAPOL 5000 PA" and "KYOWAPOL 3000 PA" manufactured by Kyowa Hakko Kogyo Co., Ltd., and " DYNACOLL 7250" manufactured by Degussa Japan Co., Ltd.

### a1-3) Polymer polyol

As the polymer polyol prepared from radically polymerizable monomers, mention may be made of polymers composed of monomers having an ethylenically unsaturated group and a hydroxy group as an indispensable component. Specific examples include those obtained by polymerizing radically polymerizable monomers, for example, hydroxyalkyl (meth)acrylates such as hydroxyethyl (meth)acrylate and hydroxypropyl (meth)acrylate with another (meth)acrylates.

The method for preparing the polymer polyol includes methods in which radically polymerizable monomers are subjected to solution polymerization or high temperature continuous polymerization.

### a2) Polyisocyanate

As the polyisocyanate, various compounds can be used as far as the compounds each have two or more isocyanate groups in molecule thereof; diisocyantes are preferable. Specific examples include p-phenylene diisocyanate, 4,4'-diphenylmethane diisocyanate, tolylene diisocyanate, 4,4'-diphenylene diisocyanate, 1,5-octylene diisocyanate, trimethylene diisocyanate, tetramethylene diisocyanate, hexamethylene diisocyanate, pentamethylene diisocyanate, 1,3-cyclopentane diisocyanate, 1,4-cyclohexane diisocyanate, 4,4'-methylene bis(cyclohexyl isocyanate), methyl 2,4-cyclohexane diisocyanate, methyl 2,6-cyclohexane diisocyanate, diphenylmethane diisocyanate, 1,4-bis(isocyanatomethyl)cyclohexane, 1,3-bis(isocyanatomethyl)cyclohexane, isophorone diisocyanate and carbodiimide-modified 4,4'-diphenylmethane diisocyanate.

Among these, alicyclic and aliphatic isocyanates are preferable because they are excellent in curability of the compositions with active energy beams and in weather resistance of the cured products. As the alicyclic or aliphatic isocyanates, hexamethylene diisocyanate and isophorone diisocyanate are preferable.

Two or more types of polyisocyanates can be used in combination, as required.

In the present invention, when the urethane prepolymer is prepared, the amount of the polyisocyanate in relation to the polyol falls within the range preferably from 1 to 3, more preferably from 1.5 to 2.5, particularly preferably from 1.8 to 2.2 in terms of the equivalence ratio of the group -NCO/the group -OH.

### a3) Method for preparing urethane prepolymer

The method for preparing the urethane prepolymer can follow the conventional methods. Examples of the methods include a method in which polyol and polyisocyanate are heated in the presence of a catalyst.

As the catalyst, the catalysts commonly used in urethanization reaction can be used; examples thereof include metal compounds and amines. Examples of the metal compounds include tin based catalysts such as dibutyltin dilaurate and dioctyltin dilaurate; lead based catalysts such as lead dioctylate; zirconium based catalysts such as K-KAT XC-4025 and K-KAT XC-6212 (manufactured by KING INDUSTRIES, INC.); aluminum based catalysts such as K-KAT XC-5217 (manufactured by KING INDUSTRIES, INC.); and titanate based catalysts such as tetra 2-ethylhexyl titanate. Examples of the amines include triethylamine, N,N-dimethylbenzylamine, triphenylamine and triethylenediamine.

It is preferable that the urethanization reaction catalyst is used in a proportion as small as possible so that no adverse effects on skin may be caused.

Additionally, when the urethane prepolymer is prepared, generally-used radical polymerization inhibitors such as hydroquinone and triethylamine can be used, as required, for the purpose of preventing gelation in the course of the reaction.

Furthermore, when the urethane prepolymer is prepared, phosphorus compounds can be blended. By blending phosphorus compounds, the action of the catalyst used in esterification and ring-opening polyaddition can be brought to a halt. If the catalyst is not deactivated, esterification reaction often occurs, when the obtained urethane prepolymer is stored in the presence of moisture or heated in the presence of moisture in a subsequent reaction, or when the resultant maleimide compound and cured film of the composition are stored in the presence of moisture, and thereby the physical properties of the composition are markedly degraded.

As the phosphorus compounds, mention may be made of inorganic or organic phosphorus compounds and the like listed in the following (a) to (e).

### (a) Phosphoric acid and the alkyl esters thereof

The phosphoric acid alkyl esters include trialkyl esters such as trimethyl phosphate, triethyl phosphate, tributyl phosphate, trinonyl phosphate, triphenyl phosphate and the like.

### (b) Organic esters of phosphonic acid

It includes dibutylbutyl phosphonate and the like.

### (c) Phosphorous acid

Phosphorous acid is used alone or in combination with other phosphorus compounds, which has the strongest effects of stabilizing hue and preventing oxidative degradation.

### (d) Organic esters of phosphorous acid

They include dibutyl hydrogen phosphite, triphenyl phosphite and the like. However, triphenyl phosphite sometimes degrades the properties of the polyester skeleton in the maleimide compound, and accordingly it is necessary to pay attention to the addition amount thereof.

### (e) Other inorganic phosphorus compounds

They include polyphosphoric acid and the like.

The amount of a phosphorus compound to be added may be appropriately set according to molecular weight of the phosphorus compound (content of phosphorus atom); generally, the amount is preferably 0.001 to 3 parts by mass, and more preferably 0.01 to 1 part by mass, in relation to 100 parts by mass of the polyester polyol. When the addition amount of a phosphorus compound is less than 0.001 part by mass, no effect resulting from the addition can be found, while when the addition amount is larger than 3 parts by mass, no further increase of the effect is expected.

### B) Maleimide/active hydrogen compound

As the maleimide/active hydrogen compound, an alcohol having a maleimide group (hereinafter referred to as a maleimide alcohol) is preferable. As the maleimide alcohol, mention may be made of the maleimide alkyl alcohols represented by the following formula (5-1) to formula (5-4).

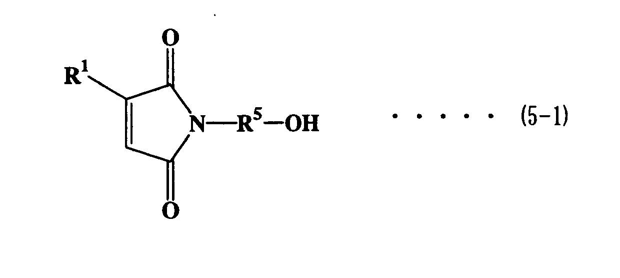

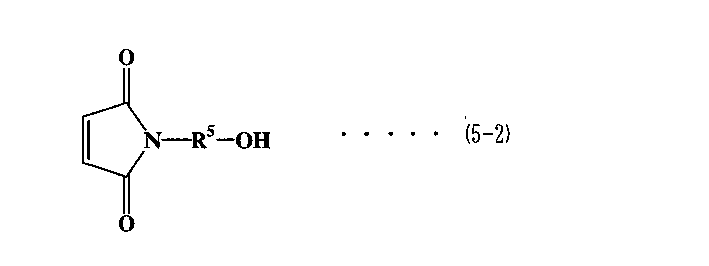

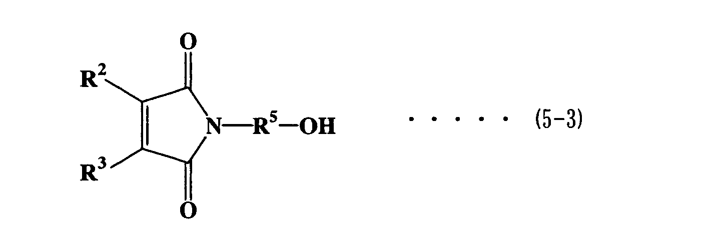

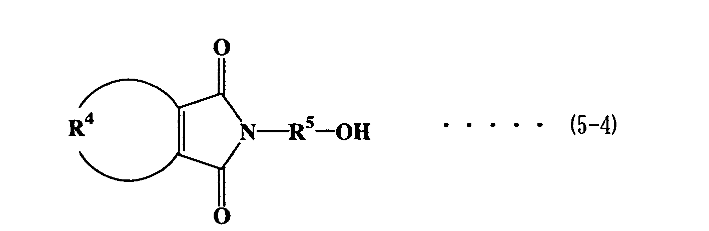

In each of formula (5-1) to formula (5-4), R⁵ represents an alkylene group, and is preferably a straight chain or branched chain alkylene group having 1 to 6 carbon atoms. In formulas (5-1), (5-3) and (5-4), R¹ to R⁴ represent the same groups as described above.

### C) Method for preparing the component (A)

As for the method for preparing the component (A), the component (A) may be prepared by reacting a urethane prepolymer and a maleimide/active hydrogen compound with each other, according to generally-employed urethanization reaction. The specific urethanization reaction includes a method similar to that described above.

In the preparation of the component (A), it is preferable that the reaction is carried out in the presence of an antioxidant for the purpose of preventing discoloration of the resultant maleimide compound.

The antioxidant includes generally used phenol based, triphosphite based and amine based antioxidants; for example, compounds described in Japanese Patent Publication Nos. S36-13738, S36-20041, S36-20042 and S36-20043.

As the phenol based antioxidant, various types can be used, and the following compounds are particularly preferable: butylhydroxytoluene, pentaerythrityl tetrakis[3-(3,5-di-t-butyl-4-hydroxyphenyl)propionate], octadecyl-3-(3,5-di-t-butyl-4-hydroxyphenyl)propionate and the like.

The phenol based antioxidant can be used in combination with zinc oxide for the purpose of enhancing the effect.

The proportion of the antioxidant to be added is preferably 0.01 to 2 parts by mass in relation to the 100 parts by mass of the urethane prepolymer. When this proportion is less than 0.01 part by mass, a sufficient effect of blending of the antioxidant cannot be obtained as the case may be, while the blending with a proportion larger than 2 parts by mass can be expected to provide no further increase of effect, and hence is disadvantageous in cost.

Additionally, when the maleimide compound is prepared, the above described phosphorus compound may be blended.

### 1-2. Compound (2)

The compound (2) is an esterification reaction product between a prepolymer having two or more carboxyl groups at terminals thereof (hereinafter simply referred to as carboxylic acid prepolymer) and a maleimide/active hydrogen compound.

The carboxylic acid prepolymer includes one prepared by use of polycarboxylic acids and polyols or polyhydric alcohols as described above.

The maleimide/active hydrogen compound includes the compounds similar to those described above.

The method for esterification reaction between the carboxylic acid prepolymer and the maleimide/active hydrogen compound may follow the method similar to that described above.

The compound (2) is preferably used as a maleimide compound that is required to be lower in viscosity.

### 1-3. Compound (3)

The compound (3) is an esterification reaction product between a prepolymer having two or more hydroxy groups at terminals thereof (hereinafter simply referred to as polyol prepolymer) and a carboxylic acid having a maleimide group (hereinafter referred to as a maleimide carboxylic acid).

The polyol prepolymer includes those as described above in connection with the polyol.

As the maleimide carboxylic acid, various types of compounds can be used, and the compounds represented by the following formulas (6-1) to (6-4) are preferable.

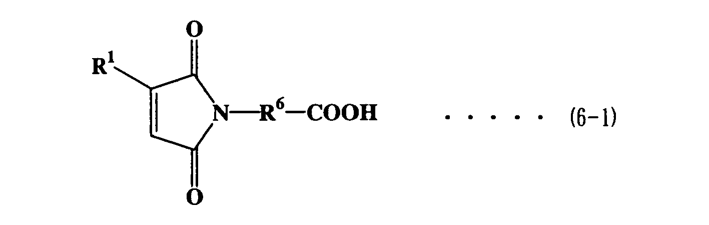

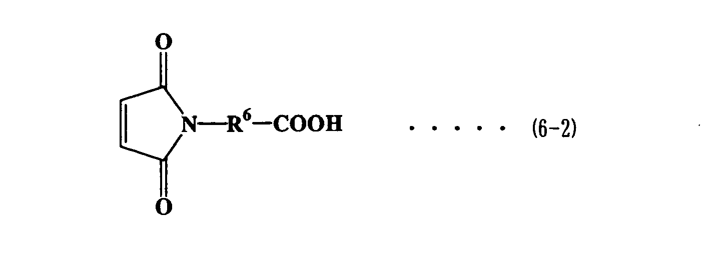

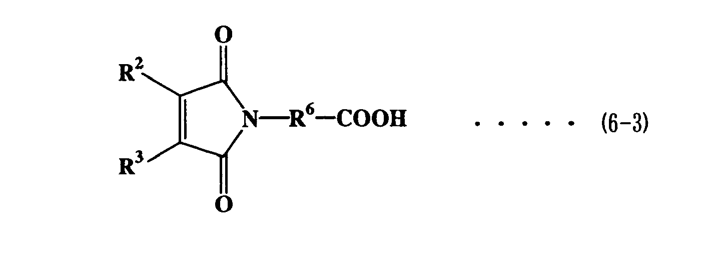

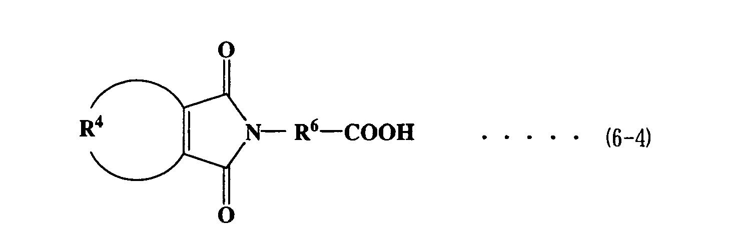

In each of formula (6-1) to formula (6-4), R⁶ represents an alkylene group, and is preferably a straight chain or branched chain alkylene group having 1 to 6 carbon atoms. In formulas (6-1), (6-3) and (6-4), R¹ to R⁴ represent the same groups as described above.

The method for esterification reaction between the polyol prepolymer and the maleimide carboxylic acid may follow the method as described above.

The compound (3) is preferably used as a maleimide compound that is required to be lower in viscosity.

### 1-4. Preferable maleimide compounds

Among the above described compounds (1) to (3), the compound (1) is preferable in that the compound (1) is faster in reaction rate, higher in yield and easier to prepare than the compounds (2) and (3). For applications where the maleimide compound is required to be low in viscosity, the compounds (2) and (3) are preferable.

The maleimide compound can adjust the viscosity and fluidity of the composition, the pressure-sensitive adhesive strength, the holding power and the tackiness of the cured film, the blending properties of the component (B) and the medicinal ingredient, and the comfortability in use and the peeling properties when skin patches are applied to the skin, by varying copolymerization composition depending upon purposes.

As the component (A) to be used in the present invention, various compounds can be used as far as the compounds have maleimide groups; the compounds having a polyether skeleton or a polyester skeleton are preferable because they are excellent in curability with active energy beams and in various adhesive performances, and excellent in water resistance of the cured films, and particularly preferable are the compounds having a polyether skeleton. The maleimide compounds having a polyether skeleton are moderate in pressure-sensitive adhesive strength, exhibit moderate adhesive performances, and cause neither pain in the patch-applied area nor damage to the horny layer of the patch-applied area even when patches are repeatedly applied to and peeled off the skin.

Preferable among the polyether skeletons are the skeletons comprising a propylene oxide unit as the indispensable component.

As the maleimide compound having a polyether skeleton comprising a propylene oxide unit as the indispensable component, may be used those comprising an ethylene oxide unit in addition to the propylene oxide unit, in which proportion of propylene oxide unit can be controlled depending upon whether an oil component or an aqueous ingredient is blended. The concrete preferable proportion is as described above in the section of polyether polyol.

Preferable as the component (A) are compounds containing two maleimide groups because such compounds provide the cured film of the composition with excellent pressure-sensitive adhesive strength and tackiness.

### 2. (B) Oil components, or water or water-soluble compounds

The composition of the present invention is blended with an oil component, or water or a water-soluble compound as the component (B). It serves to improve adhesion to the skin and to prevent damage to the skin when the skin patch is peeled off, and further serves to promote solubilization of medicinal ingredients into the composition, to work as a carrier of medicinal ingredients, and to improve transdermal absorption when medicinal ingredients described later are blended.

The choice between the oil component and water or a water-soluble compound to be blended as the component (B) may be made according to the application purpose of the skin patch, hydrophilicity and lipophilicity of the component (A) to be used, and hydrophilicity and lipophilicity of the medicinal ingredient.

### 2-1. Oil component

As the oil component, various compounds can be used as far as they exhibit oiliness.

Specific examples include: fatty acids such as myristic acid, palmitic acid, stearic acid, lauric acid, oleic acid, isostearic acid, neodecanoic acid, trimethylhexanoic acid and neoheptanoic acid; the esters of these fatty acids such as isopropyl myristate; fatty alcohols such as myristyl alcohol, cetyl alcohol, oleyl alcohol and lauryl alcohol; aliphatic pyrrolidones such as N-lauryl-2-pyrrolidone; terpenes such as 1-menthol, d-limonene and α-terpineol; alkanes such as heptane, octane, nonane and decane; and substances acting as solubilizers and transdermal absorption promoters of medicinal ingredients such as crotamiton and α-, β- and γ-cyclodextrin. Besides, mention may be made of substances for use as fragrant/refreshing materials such as L-menthol, camphor, thymol, mint oil, castor oil, fennel oil, star anise oil, cinnamon oil, oil of cloves, thiamine oil, turpentine oil, eucalyptus oil, lavender oil, lemon oil, orange oil, bergamot oil and rose oil.

### 2-2. Water or water-soluble compounds

As the water-soluble compounds, various compounds can be used as far as they exhibit water solubility.

Specific examples thereof include: alcohols such as ethanol, propanol and butanol; polyols such as propyleneglycol, ethyleneglycol, polyethyleneglycol and glycerin; amines such as diethanolamine and triethanolamine; and pyrrolidones such as N-methyl-2-pyrrolidone and 2-pyrrolidone.

Alternatively, there can be used an aqueous solution dissolving the water-soluble compound, as required. In this case, the proportion of the water-soluble compound in the aqueous solution is preferably 3 to 80% by mass.

As water or a water-soluble compound, water alone is preferably used, or an aqueous solution of the water-soluble compound is preferably used.

The component (B) is preferably a compound that has an absorbance of 1.0 or less at a wavelength of 365 nm since it provides a composition excellent in curability. Examples of such component (B) include isopropyl myristate, N-methyl-2-pyrrolidone, water, ethanol and the like.

When medicinal ingredients described later are blended, it is more preferable that two or more types of compounds are used in combination as the component (B), for the purpose of satisfactorily promoting the solubilization of medicinal ingredients into the composition, acting as a carrier of medicinal ingredients, enhancing transdermal absorption, and preventing the skin from being damaged when the skin patch is peeled off. In this case, the compound may be appropriately selected according to individual purposes on the basis of solubility of medicinal ingredients, effect on horny layer, and adjustability to appropriate pressure-sensitive adhesiveness. For example, mention may be made of a case in which a compound for facilitating dissolution of medicinal ingredients and a compound for attaining an appropriate adhesiveness to the skin are used in combination, more specifically, a case in which N-methyl-2-pyrrolidone and isopropyl myristate are used in combination.

### 3. Composition curable with active energy beam for use in skin patch

The composition of the present invention requires the components (A) and (B) as indispensable components, and is a composition liquid at ordinary temperature.

The component (A) functions as a pressure-sensitive adhesive component.

The component (A) is preferably selected from the component (I), i.e., the compound 1, or the component (II), i.e., (a) the compound 2 and (b) the compound 3 or/and the compound 4.

When the component (II) is selected as the component (A), the proportions of the components (a) and (b) are such that, in relation to 100 parts by mass of the total amount of the components (a) and (b), the contents of the components (a) and (b) are preferably 20 to 40 and 80 to 60 parts by mass, respectively, and more preferably 25 to 35 and 75 to 65 parts by mass, respectively. When the content of the component (a) is less than 20 parts by mass, the curability of the composition is lowered and the cohesion force of the cured film is lowered as the case may be. On the other hand, when the content of the component (a) exceeds 40 parts by mass, the adhesiveness and the water resistance of the cured film are often lowered.

When the compounds 3 and 4 are used in combination as the component (b), the contents of the compounds 3 and 4 are preferably 10 to 90 and 90 to 10 parts by mass, respectively, in relation to 100 parts by mass of the total amount of the compounds 3 and 4.

The blending proportions of the components (A) and (B) may be appropriately set according to purposes. When an oil component is blended as the component (B), the content of the oil component is preferably 0.1 to 50% by mass, and more preferably 1 to 30% by mass, in relation to the total amount of the components (A) and (B). When an aqueous ingredient is blended, the content of the aqueous ingredient is preferably 0.1 to 80% by mass, and more preferably 0.3 to 40% by mass, in relation to the total amount of the components (A) and (B).

The composition of the present invention is required to be liquid at ordinary temperature. By being liquid, handling including coating operation comes to be easy. On the contrary, a composition which is solid at ordinary temperature is not easy to handle, and additionally, leads to a high elastic modulus of the cured film and thus to insufficient adhesive performance.

The composition of the present invention can contain various components as required, in addition to the above described indispensable components. Now, description will be made below of the respective components.

### 3-1. Photopolymerization initiator and the like

As described above, the composition of the present invention is easily curable with active energy beams; even when cured with ultraviolet light or visible light, the composition of the present invention has an excellent curability without blending of photopolymerization initiators or with blending of a small amount of a photopolymerization initiator, but can be blended with a photopolymerization initiator or the like as required.

In the case where a photopolymerization initiator is blended, examples of the photopolymerization initiator include benzoin and its alkyl ethers, acetophenones, anthraquinones, thioxanthones, ketals, benzophenones, xanthones, acylphosphine oxides, α-diketones and the like.

Additionally, for the purpose of improving the sensitivity to active energy beams, photosensitizers can be used.

As the photosensitizer, mention may be made of benzoic acid based photosensitizers, amine based photosensitizers, and the like. These photosensitizers can be used in combination of two or more thereof. The blending proportion of these photosensitizers is preferably 0.01 to 10 parts by mass in relation to 100 parts by mass of the component (A).

As the photopolymerization initiator, benzophenones and thioxanthones are preferable because they are highly effective for improving the curing rate of the compositions.

### 3-2. Compounds having reactive unsaturated groups

For the purpose of enhancing the pressure-sensitive adhesive performance of the cured film, or for the purpose of adjusting the sensitivity of the composition, the present composition may contain a compound having a reactive unsaturated group such as (meth)acrylic monomer, (meth)acrylic oligomer and the like.

Examples of the (meth)acrylic monomer include alkyl acrylates or alkyl methacrylates (hereinafter acrylate or methacrylate will be referred to as (meth)acrylate), hydroxyalkyl (meth)acrylates, (meth)acrylates of phenol alkylene oxide adducts, mono or di(meth)acrylates of glycols, polyol poly(meth)acrylates, and poly(meth)acrylates of polyol alkylene oxide adducts.

Examples of the (meth)acrylic oligomer include urethane (meth)acrylate oligomers, polyester (meth)acrylate oligomers, and epoxy(meth)acrylate oligomers.

The proportion of the compound having a reactive unsaturated group to be added is preferably 50 parts by mass or less, and more preferably 20 parts by mass or less, in relation to 100 parts by mass of the component (A).

### 3-3. Polymers

The composition of the present invention may be supplemented with a polymer for the purpose of adjusting the viscosity before curing or adjusting the pressure-sensitive adhesive performance after curing. There is no particular limitation to the polymer concerned, and examples of the polymer include (meth)acrylate based polymers, polystyrenes and polyolefins. Among these, (meth)acrylate based polymers having a maleimide group are preferable because these polymers form, upon curing, crosslinkage with the component (A) of the present invention.

### 3-4. Other maleimide compounds

The composition of the present invention may contain a compound having one maleimide group as far as curability with irradiation of active energy beams and performance of the cured film are not damaged.

Examples of the compound concerned include a compound having one maleimide group in a skeleton as described above, a compound having a maleimide group and an ethylenically unsaturated group, and the like.

In the case where the component (A) of the present invention is originated from a polymer polyol prepared from the above described radically polymerizable monomers, the polymer polyol is often a mixture of a polymer polyol having two or more hydroxy groups in a molecule and a polymer polyol having one hydroxy group in a molecule. In this case, the resultant compound is a mixture of a compound having two or more maleimide groups and a compound having one maleimide group, and can be used as it is.

The blending proportion of the compound having one maleimide group is preferably 80 parts by mass or less and more preferably 50 parts by mass or less, in relation to 100 parts by mass of the total amount of the component (A).

### 3-5. Tackifiers

The composition of the present invention may contain a tackifier for the purpose of lowering glass transition temperature (hereinafter abbreviated as Tg) or enhancing pressure-sensitive adhesive performance of the cured film.

As the tackifier, various substances can be used; examples thereof include natural resins such as rosin based resins and terpene based resins and the derivatives thereof, and synthetic resins such as petroleum resins. Among these, preferable are those compounds which have no double bond or are small in double bond content, because they scarcely inhibit the curing with active energy beams of the composition.

The blending proportion of the tackifier is preferably 20 parts by mass or less, and more preferably 10 parts by mass or less in relation to 100 parts by mass of the component (A). When the blending proportion of the tackifier exceeds 20 parts, the viscosity of the composition becomes too high, and accordingly, the coatability is degraded.

### 3-6. Crosslinkers

The composition of the present invention may contain a crosslinker capable of reacting rapidly at ordinary temperature, for the purpose of forming crosslinkage between polymer molecules and thereby enhancing the cohesion force. Examples of the crosslinker include polyisocyanate compounds, polyoxazoline compounds, epoxy resins, aziridine compounds, polycarbodiimide resins and coupling agents.

### 3-7. Acid masking agents

When the component (A) is derived from a polyester, an acid masking agent may be blended in the composition.

Polyester is known to be hydrolyzed to lower the molecular weight thereof when it is used over a long period of time under a harsh condition such as high temperature or humidity; also in the case of skin patches, sometimes the pressure-sensitive adhesive strength is lowered depending on the storage conditions of the patches, and sometimes paste residue is left on the skin when the patches are peeled off from the skin.

In this case, blending of the acid masking agent can trap the carboxyl groups generated by the hydrolysis so that the hydrolysis can be prevented from further proceeding.

As the acid masking agent, mention may be made of carbodiimide compounds, oxazoline compounds, epoxy compounds, and the like.

The blending proportion of the acid masking agent is preferably 0.1 to 2 parts by mass in relation to 100 parts by mass of the component (A).

### 3-8. Medicinal ingredients

When the composition of the present invention is used for medical purposes and the like, medicinal ingredients may also be blended therewith.

Examples of such medicinal ingredients include: antiinflammatory agents such as salicylic acid and derivatives thereof, indomethacin, ketoprofen, flurbiprofen, ibuprofen, thiaprofenic acid, zaltoprofen, pranoprofen, loxoprofen sodium, ampiroxicam, piroxicam, meroxicam, lornoxicam, tiaramide hydrochloride, diclofenac sodium, ferbinac, bufexamac and mefenamic acid; antianginal agents such as nitroglycerin, isosorbide nitrate and pindolol; travel sickness agents such as scopolamine; cancer pain relievers such as fentanyl; hormone supplements such as estradiol; antihypertensives such as clonidine hydrochloride, methyldopa, hydralazine hydrochloride, rescinnamine, doxazosin mesilate, bunazosin hydrochloride, prazosin hydrochloride, carvedilol, arotinolol hydrochloride, labetalol hydrochloride, tilisolol hydrochloride, betaxolol hydrochloride, metoprolol tartrate, delapril hydrochloride, temocapril hydrochloride, captopril, aracepril, benazepril hydrochloride, imidapril hydrochloride, enalapril maleate, lisinopril, nifedipine, manidipine hydrochloride, benidipine hydrochloride, nisoldipine and nicardipine hydrochloride; smoking-stop aids such as nicotine; and antasthmatics such as tulobuterol hydrochloride, orciprenaline sulfate, trimetoquinol hydrochloride, terbutaline sulfate, isoproterenol sulfate, procaterol hydrochloride, theophylline, ipratropium bromide, azelastine hydrochloride, sodium cromoglicate, ibudilast, ketotifen fumarate, oxatomide and tranilast.

The blending proportion of the medicinal ingredient may be appropriately set according to purposes, and is preferably 0.1 to 20% by mass in the composition.

### 3-9. Miscellaneous

The composition of the present invention may contain a filler for the purpose of coloring and enhancing adhesive performance. Specific examples of the filler include various types of silica, dyes, calcium carbonate, magnesium carbonate, titanium dioxide, iron oxides, glass fibers, and the like.

Additionally, according to use, radical polymerization inhibitors including hydroquinone and hydroquinone monomethyl ether may be blended.

In addition to these, the following common additives used in adhesives or pressure-sensitive adhesives can be simultaneously used in the proportions usually applied: antifoamers, dyes and pigments, thickeners, lubricants, film forming aids, fillers, plasticizers, antistatic agents, textile auxiliaries, detergents, antistatic agents, level dyeing agents, dispersion stabilizers, hydrophilic resins, latexes, wetting agents, leveling improvers and the like. Additionally, the following common additives used in skin patches can be simultaneously used in the proportions usually applied: antioxidants such as dibutylhydroxytoluene and butylhydroxyanisole; preservatives such as methyl p-oxybenzoate and propyl p-oxybenzoate; and emulsifying agents.

Additionally, for the purpose of adjusting the viscosity, organic solvents can be blended as required.

Furthermore, the above described phosphorus compounds and antioxidants can be blended.

### 4. Method for producing skin patches

The composition of the present invention can be used as skin patches in various forms such as tapes, plasters and cataplasms.

The method for producing a skin patch by use of the composition of the present invention can follow the conventional methods. Examples of the preferable methods include a method in which the composition of the present invention is applied onto a substrate and the coating concerned is irradiated with an active energy beam and thereby cured.

The cured coating film thus obtained has pressure-sensitive adhesiveness and forms the plaster layer of a plaster or a cataplasm.

Examples of the substrate include paper, fabric, plastic and combinations of these. Examples of fabric include woven, nonwoven and knit fabrics. Examples of plastic include polyester, nylon, polyethylene, polypropylene, polyvinyl chloride, polyacrylate, polycarbonate, polyethylene terephthalate, and acrylonitrile/butadiene/styrene copolymer.

Examples of the coating method of the composition include roll coating, die coating and knife coating.

The coating quantity of the composition for a substrate may be appropriately chosen according to intended use; the coating quantity is preferably 5 to 200 g/m², and more preferably 10 to 100 g/m². When the coating quantity is less than 5 g/m², the pressure-sensitive adhesive strength often comes to be insufficient, while when the coating quantity exceeds 200 g/m², the active energy beam can hardly reach the deep portion and accordingly the intended performance cannot be obtained as the case may be.

After the completion of the coating process, an active energy beam is irradiated and the maleimide groups of the component (A) are thereby crosslinked to each other to increase the molecular weight, so that the cohesion force and the pressure-sensitive adhesive strength of the obtained cured film are enhanced.

The method for irradiating the active energy beam in this case may follow the method applied to the conventional pressure-sensitive adhesives curable with active energy beams. Examples of the active energy beam include visible light, ultraviolet light, X-ray and electron beam; it is preferable to use ultraviolet light, for which an inexpensive device can be used. Examples of the light source for the case where ultraviolet light is used include an ultra high pressure mercury lamp, a high pressure mercury lamp, a medium pressure mercury lamp, a low pressure mercury lamp, a metal halide lamp, a xenon lamp, an electrodeless discharge lamp and a carbon arc lamp; it is sufficient to irradiate for a few seconds to a few minutes.

Additionally, the Tg of the cured film made of the composition of the present invention is preferably -10°C or below, and more preferably -30°C or below. Herein, it should be noted that the Tg referred to in the present invention means the peak temperature of the tanδ obtained by measuring the temperature dependence by use of a dynamic viscoelasticity measurement apparatus.

The pressure-sensitive adhesive strength of the patch of the present invention is preferably 10 to 2200 g/25 mm, and more preferably 10 to 500 g/25 mm. When the pressure-sensitive adhesive strength is less than 10 g/25 mm, sometimes the pressure-sensitive adhesiveness to the skin comes to be insufficient, while when the adhesive strength exceeds 2200 g/25 mm, the adhesiveness to the skin comes to be too strong and accordingly the horny layer is damaged as the case may be.

### 5. Applications

The skin patches obtained from the composition of the present invention are high in safety and can be used for various applications.

The skin patches of the present invention can be used, as described above, in various forms such as tapes, plasters and cataplasms.

Specific examples include first aid plasters, magnetic plasters, surgical tapes, wound-healing dressing materials, tapes for sport taping and transdermal patches.

Surgical tapes are used to secure a catheter to the body. Wound-healing dressing materials are also referred to as film dressings and are used in the curing in which the wound is tightly covered for preventing the wound from being infected from the outside, simultaneously easing the pain, and keeping the wound in a wet condition. Tapes for sport taping are used for protecting and reinforcing injured portions of joints or muscles for the purpose of prevention of the injury at the time of exercise, first aid treatment, prevention of recurrence (rehabilitation), and the like.

The composition of the present invention is useful as transdermal patches, plasters and cataplasms; applications thereto will be described below in detail.

### 5-1. Transdermal patches

Transdermal patches are tape preparations used in transdermal therapeutic systems (hereinafter referred to as TTS) in which medicinal ingredients are absorbed through the skin and delivered into the body.

The composition of the present invention can be used as the material for the pressure-sensitive adhesive ingredient to be used in transdermal patches.

Examples of the medicinal ingredients of the transdermal patches include those which contain as a main component the above described medicinal ingredients such as antiinflammatory agents, antiaginal agents, travel sickness agents, cancer pain relievers, hormone supplements, antihypertensives, smoking-stop aids and antasthmatics.

The transdermal patches are preferably blended with absorption promoters for the purpose of promoting the absorption of medicinal ingredients. Examples of the absorption promoter for medicinal ingredients, which acts on the lipid of the horny layer, include, in addition to the above described oil components, sulfoxides such as dimethyl sulfoxide; amides such as urea and dimethylacetamide; amines; terpenes; and surfactants. Examples of the absorption promoter which acts as carriers of medicinal ingredients include the above described oil components and water-soluble compounds.

The composition of the present invention can be used for any of the transdermal patches including those used in reservoir-type TTS, matrix-type TTS and pressure-sensitive adhesive-type TTS.

Referring to pressure-sensitive adhesive-type TTS, production examples of transdermal patches include a method in which a composition of the present invention containing a medicinal ingredient and an absorption promoter is applied onto a substrate, then the composition is cured by irradiation with an active energy beam, and then the resulting article is subjected to cutting.

### 5-2. Plasters

The plaster is an external medicine to be used in such a way that a plaster material solid at ordinary temperature is spread onto a substrate made of paper, fabric, plastic or the like, and is brought into close contact with the skin.

The composition of the present invention can be used as a pressure-sensitive adhesive component of the plaster material used in the plaster.

The plaster material components include fats, fatty oils, fatty acid salts, waxes, resins, purified lanolin, and rubbers. The medicinal ingredients include the above described medicinal ingredients.

### 5-3. Cataplasms

The cataplasm is an external medicine to be used for wet dressing, which includes a medicinal ingredient powder and an essential oil that are spread and formed on a substrate made of fabric or the like.

The composition of the present invention can be used as a pressure-sensitive adhesive component of the plaster base material used in the cataplasm.

Referring to shaped cataplasms, production examples of cataplasms include a method in which a medicinal ingredient, an essential oil and a plaster base material are kneaded to prepare a plaster material, and the plaster material is spread onto a substrate; the resultant article is irradiated with an active energy beam to cure the composition of the present invention, and then the cured article is covered with a liner and subjected to cutting.

As the medicinal ingredients, mention may be made of the above described medicinal ingredients, in particular, derivatives of salicylic acid and the like. As the plaster base materials, mention may be made of sodium polyacrylate, gelatin, methylcellulose, polyvinyl alcohol, purified water and the like, in addition to the composition of the present invention. As the liner, mention may be made of polyester, polyethylene, polypropylene and the like.

### Effect of the Invention

The composition of the present invention is liquid at ordinary temperature, and thus is easy to handle and excellent in coatability; and even when irradiated with visible light or ultraviolet light, it has a practical crosslinking property or curability in the absence of a photopolymerization initiator, and thus is low in odor and toxicity, and safe. Additionally, the resultant cured films are not discolored but are excellent in various pressure-sensitive adhesive performances. Also, they cause neither pain in the film-applied area nor damage to the horny layer of the film-applied area even when they are repeatedly applied to and peeled off the skin, and are also excellent in water resistance.

### Best Mode for Carrying Out the Invention

The composition of the present invention only has to comprise the aforementioned components (A) and (B) as indispensable components; however, preferable as the component (A) are the following.

Specifically, preferable as the component (A) is a component which is liquid at ordinary temperature. Additionally, preferable as the component (A) is the aforementioned component (I) or (II). Preferable as the component (A) are components having a polyether skeleton and a polyester skeleton; particularly preferable is a component having a polyether skeleton. Furthermore, preferable as the component (A) is one having a number average molecular weight of 1,000 to 20,000.

Additionally, the composition preferably contains a medicinal ingredient.

### EXAMPLES

More specific description of the present invention will be made below with reference to the examples and comparative examples.

Here, it should be noted that, in the following, "%" means "% by mass."

### Production Example 1

In a flask equipped with a stirrer, a thermometer and a condenser, 270 g of a polyether polyol, namely, Adeka Polyether P-3000 (polypropyleneglycol manufactured by Asahi Denka Co., Ltd.; hereinafter referred to as P-3000) was placed at room temperature, and heated to raise the temperature up to 120°C while being stirred, and dehydrated for 1 hour under reduced pressure.

After dehydration, the dehydrated matter was cooled to 60°C, and combined with 40.4 g of isophorone diisocyanate (hereinafter referred to as IPDI) and 0.050 g of di-n-butyltin dilaurate (hereinafter referred to as DBTL) to allow the reaction to proceed for 2 hours. Thereafter, the temperature was raised up to 80°C and the reaction was further continued for 30 minutes. Then, 28.5 g of 2-hydroxyethyl citracoimide (a compound represented by the following formula (7); hereinafter referred to as CM-ETA) was added to the reaction mixture and the reaction was allowed to proceed for 2 hours to produce a maleimide compound.

The viscosity of this compound at 25°C was 26,000 mPa·s and the number average molecular weight (hereinafter abbreviated as Mn) thereof was about 3,800. This compound is referred to as M1.

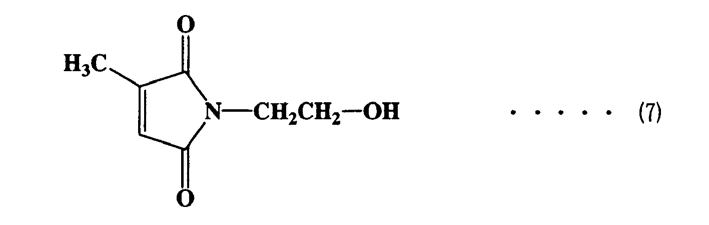

### Production Examples 2 to 9 and Comparative Production

### Example 1

Maleimide compounds were produced under the same conditions as in Production Example 1 except that the components listed in the following table were used in the amounts also listed in the following table.

The viscosity and the Mn of each of the compounds obtained are shown in Table 1.

**[Table 1]**

| | Compound name | Prepolymer | | | Maleimide alcohol | Physical properties | |
|---|---|---|---|---|---|---|---|
| | | Polyol | | Polyisocyanate | | Viscosity⁸⁾ | Mn |
| | | Name (g) | Polyol skeleton⁷⁾ | Name (g) | Name (g) | | |
| Production example 1 | M1 | P-3000 (270) | PPG Mn=3000 | IPDI (40.4) | CM-ETA (28.5) | 26,000 | 3,800 |
| Production example 2 | M2 | CM-294¹⁾ (290) | EO/PO=40/60 block copolymer Mn=2900 | IPDI (45.7) | CM-ETA (32.2) | 21,000 | 3,700 |
| Production example 3 | M3 | 70-4000²⁾ (300) | EO/PO=70/30 block copolymer Mn=4000 | IPDI (34.3) | CM-ETA (24.2) | 27,000 | 4,800 |
| Production example 4 | M4 | 80-4000³⁾ (300) | EO/PO=80/20 block copolymer Mn=4000 | IPDI (34.0) | CM-ETA (24.0) | 40,000 | 4,800 |
| Production example 5 | M5 | PR-2008⁴⁾ (270) | EO/PO=80/20 random copolymer Mn=2000 | IPDI (59.3) | CM-ETA (41.8) | 35,000 | 2,800 |
| Production example 6 | M6 | P-3000 (270) | PPG Mn=3000 | IPDI (40.4) | ML-ETA⁹⁾ (25.9) | 25,000 | 3,700 |
| Production example 7 | M7 | CM-294 (290) | EO/PO=40/60 block copolymer Mn=2900 | IPDI (45.7) | DM-ETA¹⁰⁾ (35.1) | 21,000 | 3,700 |
| Production example 8 | M8 | CM-294 (290) | Ditto | IPDI (45.7) | HT-ETA¹¹⁾ (40.5) | 22,000 | 3,700 |
| Production example 9 | M9 | 5000PA⁵⁾ (250) | Reaction product between DMPD and adipic acid Mn=5000 | IPDI (22.8) | CM-ETA (15.9) | 600,000 | 5,800 |
| Comparative production example 1 | C1 | PE-68⁶⁾ (320) | EO/PO=80/20 block copolymer Mn=9000 | IPDI (17.2) | CM-ETA (12.1) | Solid | 9800 |

The abbreviations in Table 1 have the following meanings:
1) CM-294: Polyether polyol; Adeka Polyether CM-294 manufactured by Asahi Denka Co., Ltd.
2) 70-4000: Polyether polyol; Newpol 70-4000 manufactured by Sanyo Chemical Industries, Ltd.
3) 80-4000: Polyether polyol; Newpol 80-4000 manufactured by Sanyo Chemical Industries, Ltd.
4) PR-2008: Polyether polyol; Adeka Polyether PR-2008 manufactured by Asahi Denka Co., Ltd.
5) 5000PA: Polyester polyol; KYOWAPOL 5000PA manufactured by Kyowa Hakko Kogyo Co., Ltd.
6) PE-68: Polyether polyol; Newpol PE-68 manufactured by Sanyo Chemical Industries, Ltd.
7) PPG: Polypropyleneglycol; EO: Ethylene oxide unit; PO: propylene oxide unit; DMPD: 2,4-diethyl-1,5-pentanediol.
8) Given in units of mPa·s
9) ML-ETA: A compound represented by the following formula (8). 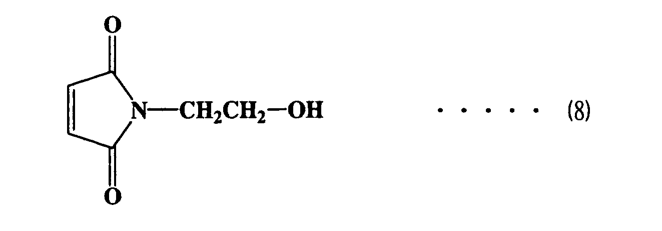
10) DM-ETA: A compound represented by the following formula (9). 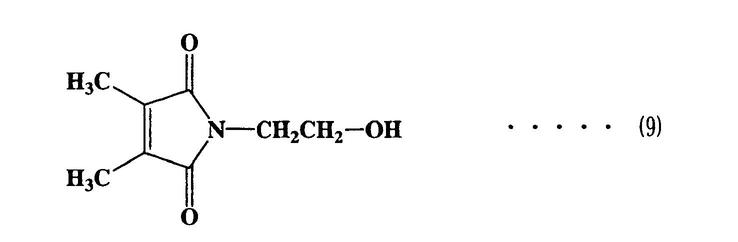
11) HT-ETA: A compound represented by the following formula (10).

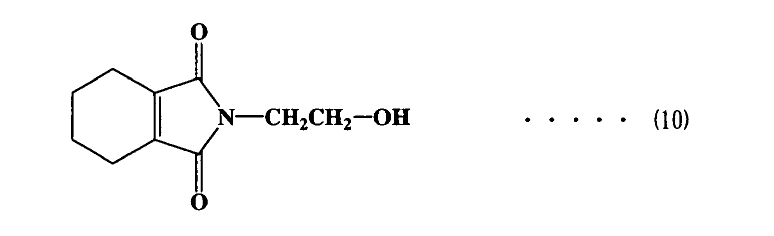

### Examples 1 to 13 and Comparative Examples 1 to 7

Using the maleimide compounds produced in the above described Production Examples and Comparative Production Example, compositions for use in skin patches were prepared by mixing the components sufficiently until the mixture became homogeneous, and then defoaming it, in accordance with the formulation shown in Table 2.

The obtained composition was applied in a thickness of 50 µm onto the surface of a 50 µm thick polyester film as the substrate sheet, and ultraviolet light (the accumulated amount of light = 1200 mJ/cm²) was irradiated using an ultraviolet irradiator equipped with a belt conveyor from the coating surface side by passing the sheet 4 times under a 120 W/cm light condensing high pressure mercury lamp (one lamp; 10 cm high) at a conveyer speed of 10 m/min., so that the composition was cured to produce a patch.

It should be noted that in each of Example 10 and Comparative Example 6, the composition was heated to 50°C and then applied, and in Comparative Example 7, C1 was heated to 80°C to melt and then applied.

In each of Examples 11 and 12, the composition was cured with an accumulated amount of light = 600 mJ/cm², and in Example 13, the composition was cured with an accumulated amount of light = 2400 mJ/cm².

The patches thus obtained each were cut into a 200 mm long and 25 mm wide specimen.

The specimens thus obtained were subjected to the following evaluations. The results are shown in Table 2.

### (1) Pressure-sensitive adhesive strength

The 180 degree peeling strength of the specimen was measured in accordance with JIS Z-0237, except that a bakelite plate was used as a test plate under the conditions of 23°C and 65% RH, in which the 100 mm long portion of the specimen was attached to the bakelite plate and pressure-bonded using a roller with a load of 2 kg; and then, the peeling strength after being allowed to stand for 30 minutes (pressure-sensitive adhesive strength 1) and the peeling strength after being allowed to stand for 24 hours (pressure-sensitive adhesive strength 2) were measured.

### (2) Tack

In conformity with JIS Z-0237 for the ball rolling method, measurements were made in the atmosphere of 23°C and 65% RH.

### (3) Bleed

The cured coating film (plaster layer) of a specimen was touched with a finger, and the bleed of the oil component, the aqueous ingredient or the like left on the finger was evaluated on the basis of the following 3 grades:
○: No bleed was found.
Δ: Slight bleed was found.
×: Bleed was found.

### (4) Paste residue

In connection with the specimen which was bonded and subjected to the peeling strength measurement after allowed to stand for 24 hours in the same manner as in above (1), the plaster layer remaining on the bakelite plate (the paste residue due to anchorage failure or cohesive failure) was observed by visual inspection and palpating with a finger to evaluate the paste residue on the basis of the following 3 grades:
○: No paste residue was found.
Δ: Slight paste residue was found.
×: Paste residue was found.

### (5) Comfortability in use for skin

A specimen was stuck on the back of a hand of each of 5 subjects, and the condition of adhesion to the skin and the comfortability for the skin were evaluated by visual inspection and palpating with a finger on the basis of the following 4 grades:
⊚: Appropriate in pressure-sensitive adhesiveness and particularly excellent in comfortability.
○: Appropriate in pressure-sensitive adhesiveness and excellent in comfortability.
Δ: Too strong in pressure-sensitive adhesive strength and thereby poor in comfortability.
×: Too weak in pressure-sensitive adhesive strength and thereby insufficient in adhesion.

### (6) Skin peeling properties

A specimen was stuck on the back of a hand of each of 5 subjects, and allowed to stand for 30 minutes; then the specimen was slowly peeled off and the presence/absence of a horny substance adhering to the specimen (damaged state of horny layer) was observed by visual inspection and evaluated on the basis of the following 4 grades:
⊚: No horny-layer was damaged.
○: Almost no horny-layer was damaged.
Δ: Some horny-layer was damaged.
×: Horny-layer was markedly damaged.

**[Table 2]**

| | Patch composition | | | Evaluation results | | | | | | |
|---|---|---|---|---|---|---|---|---|---|---|
| | Component (A) [state] (%) | Component (B) (%) | State of composition | Pressure-sensitive adhesive strength 1⁶) | Pressure-sensitive adhesive strength 2⁶⁾ | Tack | Bleed | Paste residue | Comfor tability for skin | Skin peeling properties |
| Example 1 | M1[liquid] (90) | IPM¹⁾ (10) | Liquid | 560 | 570 | 7 | ○ | ○ | ○ | ○ |
| Example 2 | M1[liquid] (70) | IPM (30) | Liquid | 120 | 140 | 6 | Δ | ○ | ⊚ | ○ |
| Example 3 | M2[liquid] (95) | OLA²⁾ (5) | Liquid | 200 | 300 | 5 | ○ | ○ | ⊚ | ⊚ |
| Example 4 | M2[liquid] (90) | OLA (10) | Liquid | 150 | 200 | 4 | ○ | ○ | ○ | ○ |
| Example 5 | M3[liquid] (70) | Water (30) | Liquid | 300 | 400 | 4 | ○ | ○ | ⊚ | ⊚ |
| Example 6 | M4[liquid] (70) | 10%EtOH ³⁾(30) | Liquid | 320 | 460 | 4 | ○ | ○ | ○ | ⊚ |
| Example 7 | M5[liquid] (70) | Water (30) | Liquid | 150 | 210 | 4 | ○ | ○ | ○ | ⊚ |
| Example 8 | M6/M7 =30/60 [liquid] (90) | IPM (10) | Liquid | 410 | 450 | 7 | ○ | ○ | ○ | ○ |
| Example 9 | M6/M8 =30/60 [liquid] (90) | IPM (10) | Liquid | 480 | 530 | 7 | ○ | ○ | ○ | ○ |
| Example 10 | M9[liquid] (90) | IPM (10) | Liquid | 1,500 | 2,100 | 9 | Δ | ○ | ○ | ○ |
| Example 11 | M1[liquid] (90) | NMP⁴⁾ (10) | Liquid | 860 | 910 | 11 | ○ | ○ | ○ | ○ |
| Example 12 | M1[liquid] (95) | NMP (5) | Liquid | 950 | 1,120 | 10 | ○ | ○ | ○ | ○ |
| Example 13 | M1[liquid] (95) | CRT⁵⁾ (5) | Liquid | 890 | 1,110 | 10 | ○ | ○ | ○ | ○ |
| Comparative example 1 | M1[liquid] (100) | - | Liquid | 860 | 820 | 12 | - | ○ | Δ | × |
| Comparative example 2 | M2[liquid] (100) | - | Liquid | 130 | 160 | 7 | - | ○ | ○ | × |
| Comparative example 3 | M3[liquid] (100) | - | Liquid | 180 | 240 | 3 | - | ○ | × | ⊚ |
| Comparative example 4 | M4[liquid] (100) | - | Liquid | 150 | 200 | 3 | - | ○ | × | ⊚ |
| Comparative example 5 | M5[liquid] (100) | - | Liquid | 35 | 67 | 3 | - | ○ | × | ⊚ |
| Comparative example 6 | M9[liquid] (100) | - | Liquid | 1,600 | 1,900 | 10 | - | ○ | Δ | × |
| Comparative example 7 | C1[solid] (100) | - | Solid | 0 | 0 | 2 or less | No adhesion No evaluation | | | |

The abbreviations in Table 2 have the following meanings, the values in parentheses denoting the absorbance (Abs) at the wavelength of 365 nm:
1) IPM: Isopropyl myristate (Abs = 0.01)
2) OLA: Oleic acid (Abs = 1.43)
3) 10% EtOH: 10% Aqueous ethanol (Abs = 0.01 or less)
4) NMP: N-Methyl-2-pyrrolidone (Abs = 0.03)
5) CRT: Crotamiton (Abs = 2.02)
6) Given in units of g/25 mm

### Examples 14 to 24 (Patch compositions containing medicinal ingredients)

Using the maleimide compounds produced in the above described Production Examples, compositions for use in skin patches were prepared by mixing the components sufficiently until the mixture became homogeneous, and then defoaming it, in accordance with the formulation shown in Table 3

The obtained composition was applied in a thickness of composition layer shown in Table 3 onto the surface of a 50 µm thick polyester film as the substrate sheet, and ultraviolet light was irradiated using an ultraviolet irradiator equipped with a belt conveyor from the coating surface side by passing the sheet under an 120 W/cm light condensing high pressure mercury lamp (one lamp; 10 cm high) at a conveyor speed of 10 m/min repeatedly until the accumulated amount of light reached the value specified in Table 3 (the accumulated amount of light per one pass = 300 mJ/cm²), so that the composition was cured to produce a patch.

The patches thus obtained each were cut into a 200 mm long and 25 mm wide specimen. The specimens thus obtained were subjected to the same evaluations as described above. The results are shown in Table 4.

**[Table 3]**

| Example No. | Patch composition | | | | |
|---|---|---|---|---|---|
| | Component (A) [state] (%) | Component (B) (%) | Medicinal ingredient (%) | State of composition | Accumulated amount of light (mJ/cm²) thickness (µm) |
| 14 | M1[liquid] (88) | NMP¹⁾ (10) | Flurbiprofen (2) | Liquid | 1,200 [50] |
| 15 | M1[liquid] (79.4) | IPM²⁾ (20) | Flurbiprofen (0.6) | Liquid | 2,400 [50] |
| 16 | M1[liquid] (84.2) | OLA³⁾ (15) | Flurbiprofen (0.8) | Liquid | 3000 [50] |
| 17 | M 1[liquid] (93) | NMP (5) | Ibuprofen (2) | Liquid | 1,200 [50] |
| 18 | M 1[liquid] (93) | NMP (5) | Ibuprofen (2) | Liquid | 600 [20] |
| 19 | M 1 [liquid] (93) | NMP (5) | Ketoprofen (2) | Liquid | 600 [20] |
| 20 | M1[liquid] (93) | NMP (5) | Indomethacin (2) | Liquid | 2,400 [20] |
| 21 | M1[liquid] (94) | NMP (5) | Diclofenac sodium (1) | Liquid | 2,700 [20] |
| 22 | M2[liquid] (75) | Water (20) | Loxoprofen sodium (5) | Liquid | 2,100 [50] |
| 23 | M1[liquid] (94) | CRT (5) | Flurbiprofen (1) | Liquid | 2,400 [50] |
| 24 | M1[liquid] (83) | NMP (10) IPM(5) | Flurbiprofen (2) | Liquid | 1,200 [50] |

The abbreviations in Table 3 have the same meanings as above.

**[Table 4]**

| Example No. | Evaluation results | | | | | | |
|---|---|---|---|---|---|---|---|
| | Pressure-sensitive adhesive strength 1¹⁾ | Pressure-sensitive adhesive strength 2¹⁾ | Tack | Bleed | Paste residue | Comfortability in use for skin | Skin peeling properties |
| 14 | 720 | 920 | 7 | ○ | ○ | ○ | ○ |
| 15 | 400 | 910 | 4 | ○ | ○ | ⊚ | ○ |
| 16 | 430 | 490 | 7 | Δ | ○ | ○ | ○ |
| 17 | 900 | 990 | 9 | ○ | ○ | ○ | ○ |
| 18 | 1,000 | 1,040 | 9 | ○ | ○ | ○ | ○ |
| 19 | 840 | 1,040 | 6 | ○ | ○ | ○ | ○ |
| 20 | 900 | 1,160 | 7 | ○ | ○ | ○ | ○ |
| 21 | 610 | 1,290 | 5 | ○ | ○ | ○ | ○ |
| 22 | 630 | 750 | 4 | ○ | ○ | ⊚ | ⊚ |
| 23 | 880 | 1,130 | 9 | ○ | ○ | ○ | ○ |
| 24 | 130 | 150 | 6 | ○ | ○ | ⊚ | ⊚ |

The abbreviation in Table 4 has the following meaning:
1) Given in units of g/25 mm.

### Industrial Applicability

The composition curable with active energy beams for use in skin patches according to the present invention can be used in various applications using skin patches.

## Claims

1. A composition curable with active energy beams for use in skin patches, which comprises (A) a compound having two or more maleimide groups and (B) an oil component, or water or a water-soluble compound, said composition being liquid at ordinary temperature.

2. The composition curable with active energy beams for use in skin patches, according to claim 1, in which said component (A) is a compound liquid at ordinary temperature.

3. The composition curable with active energy beams for use in skin patches, according to claim 1 or 2, in which said component (A) is a compound selected from the following component (I) or the following component (II):
(I): A compound having two or more maleimide groups represented by the following formula (1): 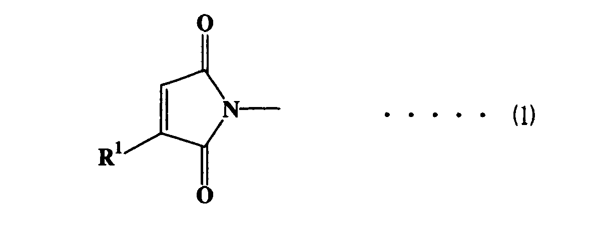 wherein R¹ represents an alkyl group, an aryl group, an arylalkyl group or a halogen atom.
(II): (a) A compound having two or more maleimide groups represented by the following formula (2), and (b) a compound having two or more maleimide groups represented by the following formula (3) or/and a compound having two or more maleimide groups represented by the following formula (4): 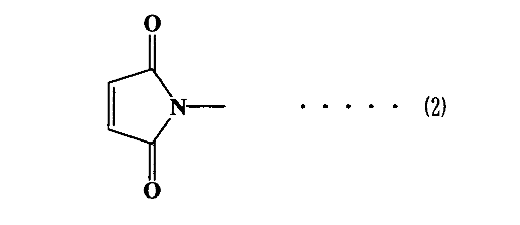 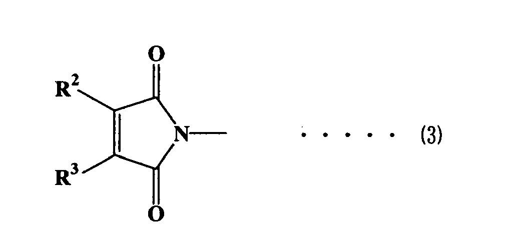 wherein R² and R³ each represent an alkyl group, an aryl group, an arylalkyl group or a halogen atom; 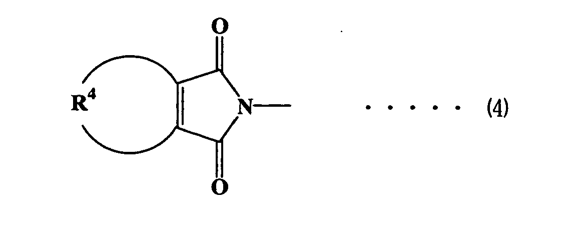 wherein R⁴ represents a propylene or butylene group which may have substituents.

4. The composition curable with active energy beams for use in skin patches, according to any one of claims 1 to 3, in which said component (A) is a compound having a polyether skeleton.

5. The composition curable with active energy beams for use in skin patches, according to any one of claims 1 to 3, in which said component (A) is a compound having a polyester skeleton.

6. The composition curable with active energy beams for use in skin patches, according to any one of claims 1 to 5, in which said component (A) has a number average molecular weight of 1,000 to 20,000.

7. The composition curable with active energy beams for use in skin patches, according to any one of claims 1 to 6, which further comprises a medicinal ingredient.

8. A skin patch which is obtained by applying the composition according to any one of claims 1 to 7 onto a substrate and then curing it by irradiation with an active energy beam.
